# EUROPEAN PATENT SPECIFICATION

(11) **EP 0 577 746 B1**
(45) Date of publication and mention of the grant of the patent: **28.02.1996**
(21) Application number: 92910303.4
(22) Date of filing: 25.03.1992
(51) Int. Cl.: C07C 67/14, C07C 69/157

(54) **IMPROVED PROCESS FOR PREPARING PHENYL CHLOROACETATE**
VERBESSERTES VERFAHREN ZUR HERSTELLUNG VON CHLORESSIGSÄURE PHENYLESTER
PROCEDE AMELIORE DE PREPARATION DE CHLOROACETATE DE PHENYLE

(30) Priority: 25.03.1991 US 674401
(43) Date of publication of application: 12.01.1994
(73) Proprietor: E.I. DU PONT DE NEMOURS AND COMPANY, Wilmington Delaware 19898 (US)
(72) Inventor: DUMAS, Donald, Joseph, Wilmington, DE 19803 (US); KRACKOV, Mark, Harry, West Chester, PA 19382 (US); SONNICHSEN, George, Carl, Wilmington, DE 19809 (US)
(74) Representative: Woodcraft, David Charles
(86) International application number: US9202236
(87) International publication number: WO9216491

(56) References cited:
- EP-A- 0 402 047
- DE-A- 1 543 537
- JOURNAL OF THE CHEMICAL SOCIETY, PERKIN TRANSACTIONS II, no. 7, July 1988, Letchworth, GB, pages 1353 - 1358; D. N. KEVILL, "Nucleophilic substitution at trgonal carbon. Part 4.Substituant effects in the reaction of aliphatic acyl chlorides with methanol and phenol in acetonitrile"

## Description

The present invention relates to an improved process for preparing phenyl chloroacetate (I) 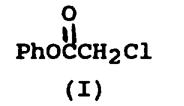 from phenol and chloroacetyl chloride without the need for a solvent and in the presence of a catalytic amount of an organic ammonium salt, phosphonium salt, amine, phosphine or amide.

Phenyl chloroacetate (I) is a useful intermediate for the preparation of acyloxyacetic acid, phenyl esters which are described in greater detail in U.S. Patent No. 4,985,180.

The process of the present invention is particularly useful since, after removing any excess chloracetyl chloride or phenol, the product may be used directly in the process of the U.S. application without further purification. The discovery is particularly important since no solvent is used giving a very economical process and extremely high volumetric efficiency. In addition, residual catalyst, if any, does not interfere with the succeeding synthesis step, and in many cases the residual catalyst also catalyzes this step.

Phenyl chloroacetate (I) is known in the art and can be prepared by a variety of methods including: the reaction of phenol with monochloroacetic acid under dehydrating conditions; the hydrolysis of 1,2,2-trichlorovinyl phenyl ether; hydrolysis of the iminoether obtained from phenol and chloroacetonitrile; and, more germane to the present application, the reaction of chloroacetyl chloride with phenol.

Journal for Praktische Chemie 1871, Vol. 4, 379 describes the preparation of phenyl.chloroacetate (I) by heating a mixture of phenol and chloroacetyl chloride. No experimental detail is provided concerning the relative amounts of the two starting materials, reaction temperature, reaction time or yield.

Chemiche Berichte 1897, Vol. 30, 1714-1715 describes the preparation of phenyl chloroacetate (I) by treating a mixture of phenol and chloroacetyl chloride in carbon disulfide with aluminum trichloride. Yield is not given.

Bulletin de la Societe Chimique de France 1899, Vol. 21, 959-960 describes the preparation of phenyl chloroacetate (I) by heating an equimolar mixture of phenol and chloroacetyl chloride. No information is provided concerning the reaction time, reaction temperature or yield.

Chemiche Berichte 1910, Vol. 43, 214 describes the preparation of phenyl chloroacetate (I) by heating a mixture of phenol and a 3-4% excess of chloroacetyl chloride at 135°C for five hours. Yield is not given.

Archiv der Pharmazie 1912, Vol. 250, 534 describes the preparation of phenyl chloroacetate (I) by treating an equimolar mixture of phenol and chloroacetyl chloride in ether with an equimolar amount of pyridine. The yield is described as good, but it is not reported.

Gazzetta Chimica Italiana 1926, Vol. 56, 764-765 describes the preparation of phenyl chloroacetate (I) by heating an equimolar mixture of phenol and chloroacetyl chloride at 130-140°C for 4-5 hours. The yield after distillation is reported to be 90-94%.

The Journal of Organic Chemistry 1959, Vol. 24, 1525 describes the preparation of phenyl chloroacetate (I) by refluxing an equimolar mixture of phenol and chloroacetyl chloride "until the evolution of HCl has ceased". Reaction times, reaction temperatures and the exact yield are not given.

Hoppe-Seyler's Zeitschrift fuer Physiologische Chemie 1981, Vol. 362, 746 describes the preparation of phenyl chloroacetate (I) by treating a mixture of phenol and a 6-7% excess of chloroacetyl chloride in benzene with a 6-7% excess of pyridine. The yield, after recrystallization, was about 40%.

The Journal of Organic Chemistry 1988, Vol. 53, 3324 describes the preparation of phenyl chloroacetate (I) from phenol and chloroacetyl chloride in dry ether in the presence of triethylamine at room temperature. No information is given as to the relative ratios of reagents. The G.C. yield is reported to be "practically quantitative".

The Journal of the Chemical Society 1965, 168-175 reports that the rate of the reaction of p-methoxyphenol with chloroacetyl chloride in acetonitrile at 70.2°C is accelerated by the presence of tetraethylammonium chloride and tetraethylammonium bromide. It is also reported that the rate of reaction of p-methoxyphenol with acetyl chloride is retarded by the presence of tetraethylammonium chloride. In the work described, the p-methoxyphenol was "always in at least a ten-fold deficit compared with acyl halide", the effect of employing smaller excesses of acid halide is not discussed. The effect of added tetraethylammonium chloride and tetraethylammonium bromide on the rate of the reaction in the absence of solvent is also not reported. In addition, no reference is made to the reaction of chloroacetyl chloride with phenol.

Bulletin de la Societe Chimique de France 1988, 383-390 reports that the rate of reaction of phenol with acetyl chloride in acetonitrile at 25°C is decelerated by the presence of tetraethylammonium chloride.

The Journal of the Chemical Society, Perkin Transactions II 1988, 1353-1358 provides rate data for the reaction of phenol with chloroacetyl chloride in acetonitrile.

Thus, the foregoing references do not discuss the preparation of phenyl chloroacetate, or substituted phenyl chloroacetates, according to a reaction of phenol, or substituted phenols, with chloroacetyl chloride in the absence of a solvent and in the presence of a catalytic amount of an organic ammonium salt, phosphonium salt, amine, phosphine, or amide.

### SUMMARY OF THE INVENTION

The present invention is an improved process for preparing phenyl chloroacetate (I). 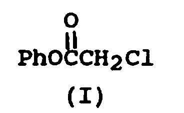 from phenol and chloroacetyl chloride in the absence of a solvent and in the presence of a catalytic amount of an organic ammonium salt, phosphonium salt, amine, phosphine or amide. The process of the invention is particularly useful since, after removing any excess chloroacetyl chloride or phenol, the product may be used directly in other processes without further purification. The discovery is particularly important in that, in comparison to known uncatalyzed solvent free processes, shorter reaction times and/or lower reaction temperatures are possible, resulting in an extremely efficient process for the production of high quality phenyl chloroacetate (I). In addition the residual catalyst does not interfere with the succeeding synthesis and in many cases these materials also catalyze that process.

### DETAILED DESCRIPTION OF THE INVENTION

According to the present invention, it is now possible to prepare phenyl chloroacetate (I) by a substantially improved and more economical process than currently known 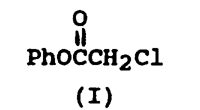 as shown in equation 1.

### Equation 1:

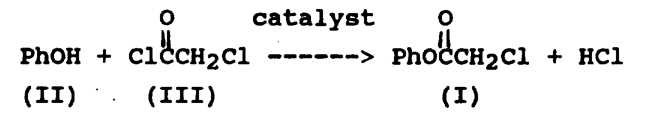

Phenyl chloroacetate (I) is prepared by reacting phenol (II) with chloroacetyl chloride (III) in the absence of a solvent and in the presence of a catalytic amount of an organic ammonium salt, phosphonium salt, amine, phosphine or amide.

The reaction of Equation 1 may be carried out at temperatures between about 50° and 200°C with temperatures between between 80° and 130°C being most preferred for convenience and overall economy. Although it is not essential, it is preferred that the process be carried out under an atmosphere of an inert gas, such as, for example, nitrogen or argon, so as to exclude atmospheric moisture and avoid possible discoloration of the product resulting from oxidation by atmospheric oxygen.

In the practice of this invention, the order of addition of the reagents is not critical; however, the evolution of by product hydrogen chloride can be vigorous, and procedures which control the evolution of hydrochloric acid will minimize the entrainment of the starting materials and product in any off gas. In a preferred embodiment, the starting materials and catalyst can be combined at a temperature between 0 and 80°C and the temperature of the reaction mass then adjusted over the course of the reaction so as to maintain an acceptable rate of hydrogen chloride evolution. In a more preferred embodiment, the phenol (II) and the catalyst can be combined and warmed to a temperature in the range of from about 80° to 130°C, and the chloroacetyl chloride (III) then added at a rate which will result in a controlled evolution of hydrogen chloride. In the practice of this invention, the optimum rate of hydrogen chloride evolution will be dependent upon the nature of the equipment employed. The selection of reaction temperatures and/or addition rates which lead to an acceptable rate of hydrogen chloride evolution is well within the means of those skilled in the art.

The reaction of Equation 1 may be carried out using any practical ratio of phenol (II) and chloroacetyl chloride (III). Preferably, between 0.95 and 1.10 molar equivalents of chloroacetyl chloride can be employed. Between 1.00 and 1.05 molar equivalents of chloroacetyl chloride is more preferred for optimum performance and economy.

In some cases, the product phenyl chloroacetate (I) may be directly processed in its crude form. Alternatively, it can be purified by conventional means. For example, excess phenol (II) and/or chloroacetyl chloride (III) may be removed by distillation either at ambient pressure or under reduced pressure. Alternatively, these excess reagents, if present, may be removed by sparging with an inert gas, such as nitrogen or argon.

Catalysts which may be employed in the process of the invention are selected from the group consisting of organic quaternary ammonium salts, quaternary phosphonium salts, amines, amine salts, phosphines, phosphine salts and amides. Quaternary arsonium salts may also be beneficially employed as catalysts for this process, although concerns about their cost and toxicity would normally preclude their use. Quaternary ammonium salts and quaternary phosphonium salts are preferred for their high catalytic activity and lower reactivity both in the process of the invention and in subsequent processing.

The amount of catalyst to be employed is between 0.001 and 0.100 moles of catalyst per mole of phenol (II). An amount of catalyst between 0.005 to 0.05 moles per mole of phenol is preferred.

Examples of quaternary ammonium salts and quaternary phosphonium salts which may be used as catalysts in practicing this invention include, but are not limited to, tetramethylammonium bromide, tetramethylammonium chloride, tetramethylammonium hydrogen sulfate, tetramethylammonium sulfate, tetramethylammonium iodide, tetraethylammonium bromide, tetraethylammonium chloride, tetraethylammonium hydrogen sulfate, tetraethylammonium iodide, tetrapropylammonium bromide, tetrapropylammonium chloride, tetrapropylammonium hydrogen sulfate, tetrapropylammonium iodide, methyltriethylammonium bromide, methyltriethylammonium chloride, methyltriethylammonium hydrogen sulfate, methyltriethylammonium iodide, methyltripropylammonium bromide, methyltripropylammonium chloride, methyltripropylammonium hydrogen sulfate, methyltripropylammonium iodide, methyltributylammonium bromide, methyltributylammonium chloride, methyltributylammonium hydrogen sulfate, methyltributylammonium iodide, tetrabutylammonium fluoride, tetrabutylammonium dihydrogenphosphate, tetrabutylammonium bromide, tetrabutylammonium chloride, tetrabutylammonium hydrogen sulfate, tetrabutylammonium methanesulfonate, tetrabutylammonium para-toluene sulfonate, methyltrioctylammonium bromide, methyltrioctylammonium chloride, methyltrioctylammonium iodide, methyltridecylammonium chloride, octadecyltrimethylammonium bromide, Aliquat® 336-PTC, Arosurf® PT 40, Arosurf® PT 41, Arosurf® PT 50, Arosurf® PT 61, Arosurf® PT 62, Arosurf® PT 64, Arosurf® PT 71, Adogen® 464, hexadecyltrimethylammonium bromide, hexadecyltrimethylammonium chloride, hexadecyltrimethylammonium para-toluene sulfonate, hexadecyltributylammonium bromide, dimethylethylhexadecylammonium bromide, tetraoctylammonium bromide, tetradodecylammonium bromide, didecyldimethylammonium bromide, dimethyldistearylammonium methanesulfonate, myristyltrimethylammonium bromide, hexadecylpyridinium bromide, hexadecylpyridinium chloride, benzyltrimethylammonium bromide, benzyltrimethylammonium chloride, benzyltriethylammonium bromide, benzyltriethylammonium chloride, benzyltributylammonium bromide, benzyldimethylhexadecylammonium chloride, benzyldimethyldodecylammonium chloride, (diisobutylphenoxyethoxyethyl)dimethylbenzylammonium chloride, phenyltrimethylammonium bromide, phenyltrimethylammonium chloride, tetrabutylphosphonium bromide, tetrabutylphosphonium chloride, hexadecyltributylphosphonium bromide, dodecyltricyclohexylphosphonium bromide, tetraphenylphosphonium bromide, tetraphenylphosphonium chloride, methyltriphenylphosphonium bromide, methyltriphenylphosphonium iodide, N-alkyl-4-(N',N'-dialkylamino)pyridinium salts, such as those described by D.J. Brunelle and D.A. Singleton in Tetrahedron Letters 1984, Vol. 25, 3383-3386, the teachings of which are incorporated herein by reference, bis-aminopyridinium salts, such as those described by D.J. Brunelle in U.S. Patent 4,595,760, the teachings of which are incorporated herein by reference, N,N,N-trialkyl-2-oxo-2-phenoxyethanaminium halides, which may be prepared by the method described by S. E. Drewes, H. E. M. Magojo and J. Gliemann in Hoppe-Seyler's Zeitschrift fuer Physiologische Chemie 1981, Vol. 362, 745-753 or modifications thereof, and (2-oxo-2-phenoxyethyl)triarylphosphonium halides, which may be prepared by the method described by H. Kunz and H. Kauth in Zeitschrift fuer Naturforschung 1979, Vol. 34b, 1737-1744 or modifications thereof.

Quaternary ammonium and phosphonium halides are preferred catalysts for their high catalytic activity. Tetramethylammonium chloride has been found to be a particularly economical catalyst for this process.

The amines and amine salts which may be employed in the process of the invention include, optionally substituted primary, secondary, or tertiary aliphatic amines, optionally substituted primary, secondary or tertiary aromatic amines, pyridine, substituted pyridines and their salts. Preferred catalysts from this group include trialkyl amines and dialkylaminopyridines and their hydrogen halide salts. Examples of amines and amine salts which may be employed include, but are not limited to, trimethylamine, trimethylamine hydrochloride, triethylamine, triethylamine hydrochloride, triethylamine hydrofluoride, tripropylamine, tributylamine, pyridine, pyridine hydrobromide, and 4-dimethylamino pyridine.

The phosphines which may be employed as catalysts in the process of the invention include trialkylphosphines and triarylphosphines, particularly tertiary phosphines such as triphenylphosphine, trimethylphosphine, triethylphosphine, tripropylphosphine and tributylphosphine.

The amides which may be employed as catalysts in the process include aliphatic amides, benzamides and cyclic amides, such as, for example, dimethylformamide, dimethylacetamide, and 1-methyl-2-pyrrolidinone.

The process of the invention can be seen in the following examples.

### EXAMPLE 1

### Preparation of Phenyl Chloroacetate Using Tetramethylammonium Chloride as Catalyst

A mixture of 110 g (1.17 mole) phenol and 2.62 g (0.024 mole, 2 mole %) of tetramethylammonium chloride was heated to 125°C and 137 g (1.21 mole, 1.03 equivalents) of chloroacetyl chloride was added over one hour with stirring. The reaction was held at 125°C for an additional hour after the addition of the chloroacetyl chloride was complete. The reaction solution was then heated to 135°C and sparged with nitrogen for 45 minutes. The product, which crystallized upon cooling, weighed 192.8 g. A sample of the product was dissolved in 10% ethanol/ethylacetate and analyzed by gas chromatography (GC) using a 30 meter DB-1 capillary column with FID detector and using the following temperature program: immediate heating at 10°C/min from an initial temperature of 40°C to 200°C followed by a 20°C/min temperature increase to 250°C and a 40 minute hold at 250°C. This GC analysis indicated the presence of 0.04% chloroacetyl chloride (based on detected ethylchloroacetate), 0.06% phenol and no detectable chloroacetic acid. Assuming that all of the catalyst was also present in the product, the purity was calculated to be 98.5% and the yield calculated to be 95%.

### EXAMPLES 2-16

As outlined below, the procedure given in Example 1 for the preparation of phenyl chloroacetate was followed except that the sparging step was omitted and different catalysts were employed in each Example.

A mixture of 11.0 g (0.117 mole) of phenol and 2 mole % catalyst was heated to 125°C and 13.7 g (0.121 mole, 1.03 equivalents) of chloroacetyl chloride added over one hour with stirring. The reaction was held at 125°C for one hour after the completion of the addition. A sample was then removed, diluted with ethyl acetate and analyzed by gas chromatography (GC) using a 15 meter DB-1 capillary column with FID detector and using the following temperature program: 5 minutes at 80°C, followed by a 10°C/min temperature increase to 200°C, and finally a 3 minute hold at 200°C. All components with retention times greater than 1.5 minutes were integrated. In every example phenol and phenyl chloroacetate accounted for greater than 96% of the area of the integrated components. In general the phenol and phenyl chloroacetate accounted for greater than 98.5% of the integrated products with the exception of Examples 7, 13, 14 and 16 in which between 2.0 and 3.5% of other components, believed to be derived from the catalysts, were also noted.

The mole % conversion of phenol to phenyl chloroacetate was calculated using the formula 100[(mole % phenyl chloroacetate)/[(mole % phenol)+(mole % phenyl chloroacetate)]]. The mole % phenol and mole % phenyl chloroacetate were determined by correcting the raw GC data using the relative molar response factor for phenol and phenyl chloroacetate. The relative molar response factor was obtained by analyzing a set of standards made up from phenol and distilled phenyl chloroacetate. The results are shown in Table 1. All numbers are rounded to the nearest 0.1%.

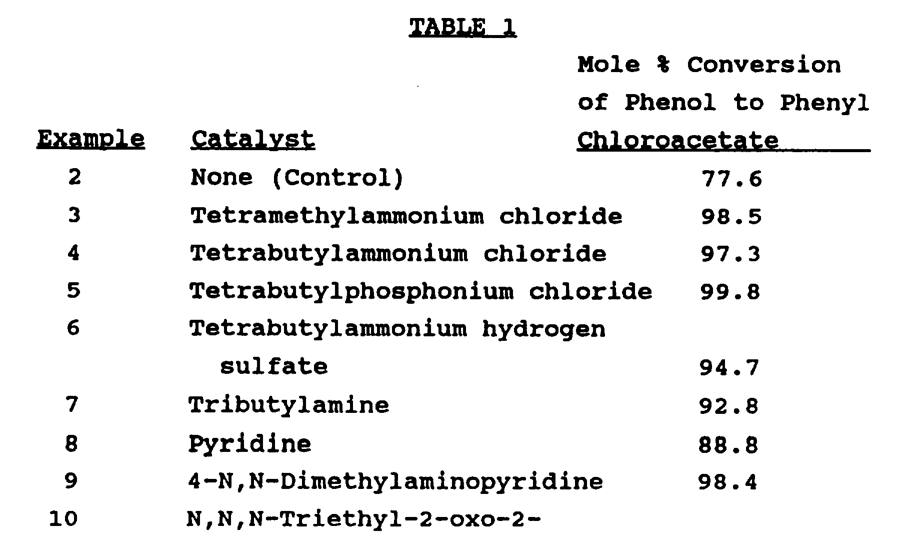

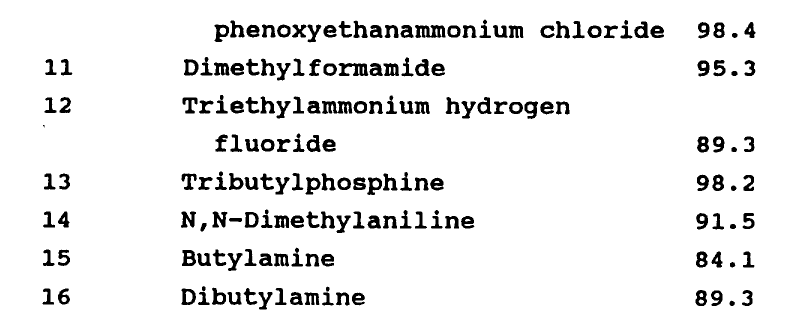

## Claims

1. A process for preparing phenyl chloroacetate which comprises reacting phenol with chloroacetyl chloride in the absence of a solvent and in the presence of a catalyst selected from the group consisting of organic ammonium salts, phosphonium salts, amines, phosphines and amides in which the molar ratio of catalyst to phenol is between 0.001 and 0.100.

2. The process of Claim 1 in which the catalyst is selected from the group consisting of quaternary ammonium salts, quaternary phosphonium salts, tertiary amines and their hydrogen halide salts, dialkylaminopyridines and their hydrogen halide salts, tertiary phosphines and amides.

3. The process of Claim 2 in which the chloroacetyl chloride is added to a mixture of the phenol and the catalyst at a temperature from about 80 to 130°C.

4. The process of Claim 3 in which the catalyst is a quaternary ammonium salt or a quaternary phosphonium salt.

5. The process of Claim 4 in which the catalyst is a quaternary ammonium halide or a quaternary phosphonium halide.

6. The process of Claim 5 in which the catalyst is tetramethylammonium chloride.

7. The process of Claim 5 in which the catalyst is tetrabutylphosphonium chloride.

8. The process of Claim 5 in which the catalyst is a N,N,N-trialkyl-2-oxo-2-phenoxyethanammonium halide.

## Patentansprüche

1. Verfahren zur Herstellung von Phenylchloracetat, welches die Umsetzung von Phenol mit Chloracetylchlorid in Abwesenheit eines Lösungsmittels und in Gegenwart eines Katalysators, ausgewählt aus der Gruppe, bestehend aus organischen Ammioniumsalzen, Phosphoniumsalzen, Aminen, Phosphinen und Amiden, bei dem das Molverhältnis von Katalysator zu Phenol zwischen 0,001 und 0,100 liegt, umfaßt.

2. Verfahren nach Anspruch 1, bei dem der Katalysator ausgewählt wird aus der Gruppe, bestehend aus quaternären Ammoniumsalzen, quaternären Phosphoniumsalzen, tertiären Aminen und ihren Halogenwasserstoffsalzen, Dialkylamino-pyridinen und ihren Halogenwasserstoffsalzen, tertiären Phosphinen und Amiden.

3. Verfahren nach Anspruch 2, bei dem das Chloracetylchlorid zu einem Gemisch aus dem Phenol und dem Katalysator bei einer Temperatur von etwa 80 bis 130 °C zugegeben wird.

4. Verfahren nach Anspruch 3, bei dem der Katalysator ein quaternäres Ammoniumsalz oder ein quaternäres Phosphoniumsalz ist.

5. Verfahren nach Anspruch 4, bei dem der Katalysator ein quaternäres Ammoniumhalogenid oder ein quaternäres Phosphoniumhalogenid ist.

6. Verfahren nach Anspruch 5, bei dem der Katalysator Tetramethylammoniumchlorid ist.

7. Verfahren nach Anspruch 5, bei dem der Katalysator Tetrabutylphosphoniumchlorid ist.

8. Verfahren nach Anspruch 5, bei dem der Katalysator N,N,N-Trialkyl-2-oxo-2-phenoxyethanammoniumhalogid ist.

## Revendications

1. Procédé pour préparer du chloroacétate de phényle, qui comprend la réaction de phénol avec du chlorure de chloroacétyle en l'absence d'un solvant et en présence d'un catalyseur choisi dans le groupe constitué des sels d'ammonium, sels de phosphonium, amines, phosphines et amides organiques, dans lequel le rapport molaire du catalyseur au phénol est entre 0,001 et 0,100.

2. Procédé selon la revendication 1, dans lequel le catalyseur est choisi dans le groupe constitué des sels d'ammonium quaternaire, des sels de phosphonium quaternaire, des amines tertiaires et de leurs sels avec des halogénures d'hydrogène, des dialkylaminopyridines et leurs sels avec des halogénures d'hydrogène, des phosphines tertiaires et des amides.

3. Procédé selon la revendication 2, dans lequel le chlorure de chloroacétyle est ajouté à un mélange du phénol et du catalyseur à une température d'environ 80 à 130 °C.

4. Procédé selon la revendication 3, dans lequel le catalyseur est un sel d'ammonium quaternaire ou un sel de phosphonium quaternaire.

5. Procédé selon la revendication 4, dans lequel le catalyseur est un halogénure d'ammonium quaternaire ou un halogénure de phosphonium quaternaire.

6. Procédé selon la revendication 5, dans lequel le catalyseur est le chlorure de tétraméthylammonium.

7. Procédé selon la revendication 5, dans lequel le catalyseur est le chlorure de tétrabutylphosphonium.

8. Procédé selon la revendication 5, dans lequel le catalyseur est un halogénure de N,N,N-trialkyl-2-oxo-2-phénoxyéthanammonium.
